(19) Europäisches Patentamt / European Patent Office / Office européen des brevets

(11) **EP 3 334 340 B1**

(12) **EUROPEAN PATENT SPECIFICATION**

(45) Date of publication and mention
of the grant of the patent:
**05.06.2024   Bulletin 2024/23**

(51) International Patent Classification (IPC):
***A61B 5/083*** (2006.01)      ***A61B 5/00*** (2006.01)

(21) Application number: **16757730.3**

(52) Cooperative Patent Classification (CPC):
**A61B 5/0836; A61B 5/725;** A61M 2230/432

(22) Date of filing: **04.08.2016**

(86) International application number:
**PCT/IB2016/054702**

(87) International publication number:
**WO 2017/025869 (16.02.2017 Gazette 2017/07)**

(54) **SIMPLIFIED DISPLAY OF END-TIDAL CO2**

VEREINFACHTE ANZEIGE VON ENDEXPIRATORISCHEM CO2

DISPOSITIF D'AFFICHAGE SIMPLIFIÉ DE CO2 EN FIN D'EXPIRATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:   **10.08.2015   US 201562203090 P**

(43) Date of publication of application:
**20.06.2018   Bulletin 2018/25**

(73) Proprietor: **Koninklijke Philips N.V.**
**5656 AG Eindhoven (NL)**

(72) Inventors:
• **ORR, Joseph**
  **5656 AE Eindhoven (NL)**
• **BREWER CATES, Lara Marie**
  **5656 AE Eindhoven (NL)**

(74) Representative: **Philips Intellectual Property & Standards**
**High Tech Campus 52**
**5656 AG Eindhoven (NL)**

(56) References cited:
**EP-A1- 1 972 356     US-A1- 2014 330 155**

**Description**

FIELD

The following relates generally to the capnography arts and related arts.

BACKGROUND

A capnography device monitors the concentration or partial pressure of carbon dioxide ($CO_2$) in respiratory gases. A common capnography parameter is the end-tidal $CO_2$ ($etCO_2$) which conceptually is the $CO_2$ partial pressure at the end of the exhalation phase. However, since this is usually the largest observed $CO_2$ partial pressure in the breathing cycle, $etCO_2$ is clinically defined as the maximum observed $CO_2$ partial pressure over the breathing cycle. The $etCO_2$ is commonly presented as a partial pressure ($PetCO_2$) or as a percentage value.

The $etCO_2$ parameter measured by capnography is commonly employed as a measurable surrogate for the maximum carbon dioxide partial pressure at the alveoli of the lungs. Knowledge of the maximum alveolar $CO_2$ partial pressure, in turn, is useful for diagnosing the state of the pulmonary and cardiopulmonary systems, and accordingly has substantial value for clinical diagnosis and patient monitoring. A stable $etCO_2$ trend line indicates stable respiration, while if the $etCO_2$ is trending downward over time this can indicate respiratory deterioration, adverse reaction to medication, impact of anesthesia or sedation, or so forth.

However, the $etCO_2$ measured by capnography is often noisy, and can vary significantly from breath to breath. The capnography $etCO_2$ can vary with changes in breathing pattern, when the patient engages in talking, coughs, or so forth.

United States Patent Application Publication Number US 2014/0330155A discloses concepts for monitoring respiratory stability and/or effective.

European Patent Application Publication Number EP 1 972 356 A1 presents a method for monitoring a patient's breathing action response to changes in a ventilator applied breathing support.

The following discloses new and improved systems and methods that address the above referenced issues, and others.

SUMMARY

According to an aspect of the invention, there is provided a capnograph device according to claim 1. In some embodiments the sliding window maximum operation employs a sliding time window whose duration is at least 30 seconds. In some embodiments performing the sliding window maximum operation comprises computing $etCO_2(t) = \max([CO_2])|_{W(t)}$ where $t$ denotes time, $[CO_2]$ is the capnogram signal (40) and $W(t)$ is a sliding time window. The capnograph device may be a sidestream or mainstream capnograph device.

In another aspect, there is provided a non-transitory storage medium storing instructions readable and executable by an electronic processor to perform a capnography method according to claim 11.

One advantage resides in providing an end-tidal carbon dioxide ($etCO_2$) value that more accurately approximates the maximum alveolar carbon dioxide level.

Another advantage resides in providing $etCO_2$ with reduced noise compared with end-tidal $CO_2$ determined on a breath-by-breath basis.

Another advantage resides in providing $etCO_2$ that both (1) more accurately approximates the maximum alveolar carbon dioxide level and (2) has reduced noise compared with end-tidal $CO_2$ determined on a breath-by-breath basis.

Another advantage resides in providing $etCO_2$ with reduced systematic error.

A given embodiment may provide none, one, two, more, or all of the foregoing advantages, and/or may provide other advantages as will become apparent to one of ordinary skill in the art upon reading and understanding the present disclosure.

BRIEF DESCRIPTION OF THE DRAWINGS

The invention is defined in the appended claims.

The drawings are only for purposes of illustrating the preferred embodiments and are not to be construed as limiting the invention.

FIGURE 1 diagrammatically illustrates a capnograph device including improved end-tidal carbon dioxide ($etCO_2$) calculation as disclosed herein.

FIGURE 2 diagrammatically illustrates performing a sliding window maximum operation on a capnogram to compute

etCO$_2$.

FIGURES 3 and 4 plot end-tidal CO$_2$ data computed on a breath-by-breath basis (FIGURE 3) and by using a sliding window maximum operation (FIGURE 4).

DETAILED DESCRIPTION

The trend of etCO$_2$ is difficult to evaluate when the patient is spontaneously breathing and the breaths are not uniform in size. During spontaneous or pressure supported ventilation, the etCO$_2$ as measured by a capnograph device can vary significantly, for example when the patient talks, coughs, suffers from sleep apnea or drug induced airway obstruction or experiences acute respiratory depression after anesthesia for a medical procedure. It is not physiologically possible for the alveolar CO$_2$ partial pressure to change as quickly as the etCO$_2$ changes observed by capnography with breaths of varying size.

An apparent solution is to smooth the etCO$_2$ trend line using a low pass filter or the like to remove the noise. However, it is recognized herein that this approach has significant disadvantages in the case of etCO$_2$ measured by capnography. This is because, as recognized herein, clinical conditions and physiological events that introduce noise into the etCO$_2$ measurement tend to systematically reduce the etCO$_2$ as measured by the capnograph device. For example, if the volume of the breath is too small to completely flush out the airway dead volume, the measured etCO$_2$ will be reduced. Similarly, if the lungs contain parallel (alveolar) dead volume, the etCO$_2$ measured by capnography will again be reduced. If supplemental oxygen is being administered to the patient, it can combine with the exhaled gas and, yet again, reduce the etCO$_2$ reading produced by capnography.

A common clinical application of etCO$_2$ measurement by capnography is to provide an accurate, measurable surrogate for the maximum alveolar CO$_2$ partial pressure which is not directly measurable. However, each of the foregoing etCO$_2$ noise sources causes a reduction in the etCO$_2$ value measured by capnography, so as to systematically deviate below the alveolar maximum CO$_2$ partial pressure. When the etCO$_2$ measured by capnography is viewed as a surrogate for the alveolar maximum CO$_2$ partial pressure, these "noise" sources are therefore not true noise sources that introduce random error. Rather, these "noise" sources are sources of systematic error, in that they systematically cause the etCO$_2$ measured by capnography to read too low when compared with the (not readily measured) gold standard of the alveolar maximum CO$_2$ partial pressure.

When viewed in light of the foregoing insights, a low pass filter or other smoothing mechanism designed to remove noise, i.e. random error, is not appropriate for improving the etCO$_2$ values measured by capnography. Rather, the appropriate improvement should preferentially display the maximum observed CO$_2$ over a relatively long period of time (e.g. encompassing around 10-30 breaths), as this is more likely to present etCO$_2$ values that accurately reflect the maximum alveolar CO$_2$. In some illustrative embodiments, the following processing is disclosed. At a fixed sampling time interval $T_S$, e.g. 5-15 seconds in some embodiments, the maximum expired CO$_2$ measured over a time window $W$ of longer interval $T_W$, e.g. 30 seconds-to-3 minutes in some embodiments, and 1-2 minutes in some embodiments, is identified. These maximum samples obtained at the sampling rate ($1/T_S$) form a sampled signal representing the etCO$_2$, with successive data points (samples) of the signal spaced apart by the sampling interval $T_S$. Optionally, this etCO$_2$ signal is smoothed, for example using a low-pass filter, to remove spurious samples (these are true noise, i.e. are expected to constitute random error).

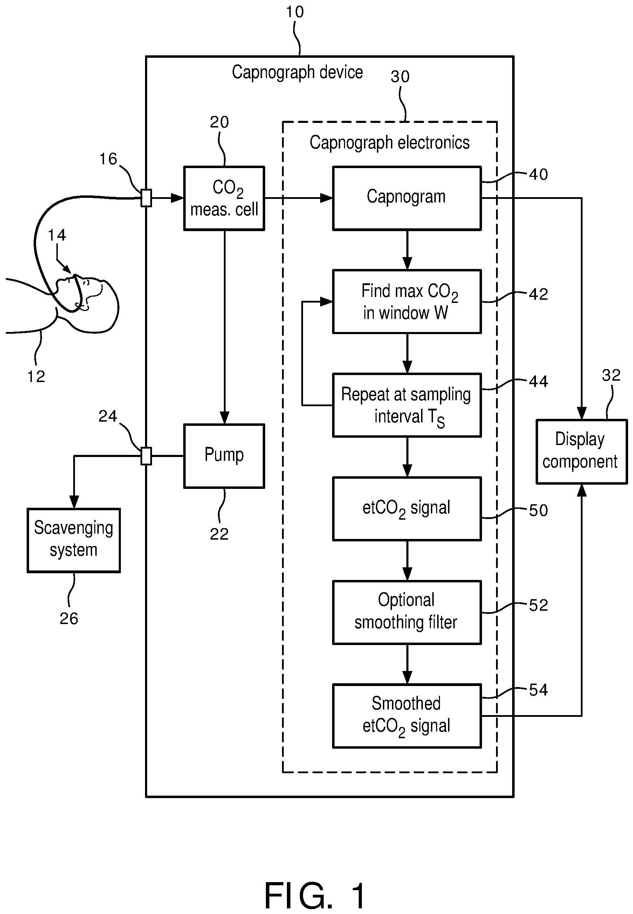

With reference to FIGURE 1, an illustrative capnograph device **10** employing such etCO$_2$ signal generation is diagrammatically shown. As shown in FIGURE 1, during operation the capnograph device **10** is connected with a patient **12** by a suitable patient accessory, such as a nasal cannula **14** in the illustrative example, or by an airway adaptor or so forth. The patient accessory **14** may optionally include one or more ancillary components, such as an air filter, water trap, or the like (not shown). In the illustrative capnograph **10,** respired air is drawn from the patient accessory **14** into a capnograph air inlet **16** and through a carbon dioxide (CO$_2$) measurement component or cell **20** by an air pump **22.** The air is then discharged via an air outlet **24** of the capnograph **10** to atmosphere or, as in the illustrative embodiment, is discharged through the air outlet **24** into a scavenging system **26** to remove an inhaled anesthetic or other inhaled medicinal agent before discharge into the atmosphere. The CO$_2$ measurement component or cell **20** may, for example comprise an infrared optical absorption cell in which carbon dioxide in the respired air drawn from the patient accessory **14** produces absorption that is detected by an infrared light source/detector assembly.

The illustrative capnograph device **10** has a sidestream configuration in which respired air is drawn into the capnograph device **10** using the pump **22,** and the CO$_2$ measurement cell **20** is located inside the capnograph device **10.** That is, the sidestream capnograph device **10** includes, as a unit, the carbon dioxide measurement component **20,** the electronic processor **30,** and the pump **22** connected to draw respired air though the carbon dioxide measurement component **20.** The sidestream configuration is suitably used for a spontaneously breathing patient, i.e. a patient who is breathing on his or her own without assistance of a mechanical ventilator. In an alternative configuration, known as a mainstream configuration (not illustrated), the CO$_2$ measurement cell is located externally from the capnograph device housing, typically as a CO$_2$ measurement cell patient accessory that is inserted into the "mainstream" airway flow of the patient. Such a mainstream configuration may, for example, be employed in conjunction with a mechanically ventilated patient in which the $CO_2$ measurement cell patient accessory is designed to mate into an accessory receptacle of the ventilator unit, or is installed on an airway hose feeding into the ventilator. The disclosed approaches for calculating $etCO_2$ are readily applied either in conjunction with a sidestream capnograph device (as in the illustrative example of FIGURE 1) or in conjunction with a mainstream capnograph device.

With continuing reference to FIGURE 1, the capnograph device **10** (in either the illustrative sidestream configuration or in the alternative mainstream configuration) includes capnograph electronics **30** which provide power and control for operating the $CO_2$ measurement cell **20** and (in the sidestream configuration) the pump **22**. Note that the power and control links are not illustrated in diagrammatic FIGURE 1. The capnograph electronics **30** additionally perform processing of the $CO_2$ signal output by the $CO_2$ measurement cell **20,** as diagrammatically indicated in FIGURE 1 and as described herein. Clinical data output by the capnograph **10,** such as a capnogram and $etCO_2$ signal, are displayed on a display component **32,** stored in an electronic medical record (EMR) or the like, or otherwise utilized. The display component **32** may be a component of the capnograph or, as illustrated in FIGURE 1, the display component **32** may be an external display component connected to the capnograph **10.** For example, the external display component **32** may be a multi-function bedside patient monitor and/or a nurses' station patient monitor or so forth. It will be further appreciated that the capnograph may include numerous other components not illustrated in simplified diagrammatic FIGURE 1, such as a pressure gauge, flow meter, and so forth.

The capnograph electronics **30** may be variously implemented, such as by a suitably programmed electronic processor, e.g. a microprocessor or microcontroller of the capnograph **10.** While a single electronics unit **30** is illustrated, it is alternatively contemplated to employ various combinations of electronics, for example different electronic components may be operatively interconnected to implement a pump power supply, infrared light source power supply (for the $CO_2$ measurement cell **20**), analog-to-digital conversion circuitry (to sample the infrared light detector of the $CO_2$ measurement cell **20**), and so forth. Still further, it is contemplated for the capnograph to output the capnogram ($CO_2$ versus time signal) without the disclosed $CO_2$ signal processing and for that processing to be performed by suitably programmed electronics in another device (for example, the computer of a nurses' station that receives the capnogram signal). It will be still further appreciated that the $CO_2$ signal processing disclosed herein as being performed by the capnograph electronics **30** may be embodied by a non-transitory storage medium storing instructions that are readable and executable by the microprocessor, microcontroller, or other electronic processor to perform the disclosed $CO_2$ signal processing including the $etCO_2$ calculation employing approaches disclosed herein. Such non-transitory storage media may, by way of non-limiting illustration, include a hard disk drive or other magnetic storage medium, a flash memory, read-only memory (ROM) or other electronic storage medium, an optical disk or other optical storage medium, various combinations thereof, or so forth.

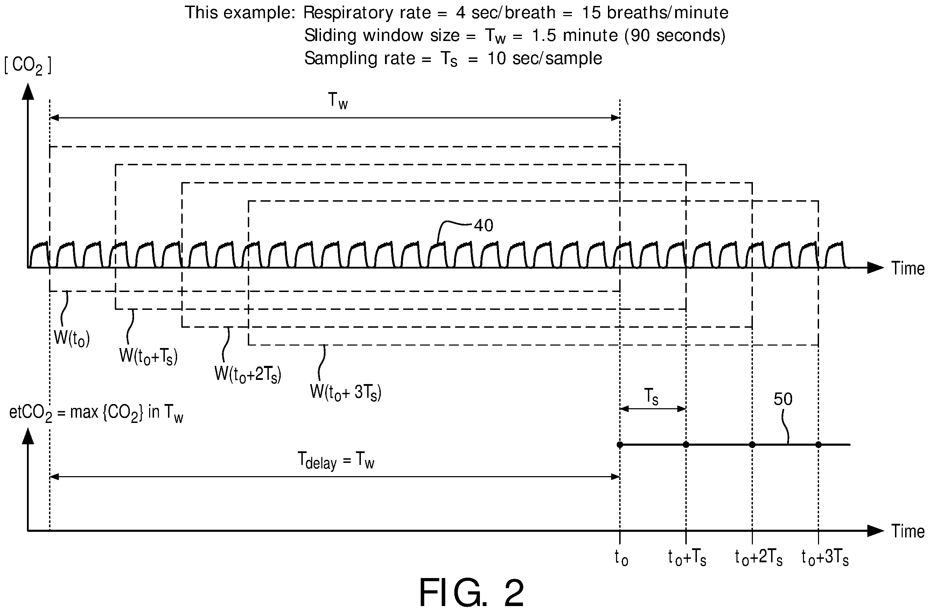

With continuing reference to FIGURE 1 and with further reference to FIGURE 2, an illustrative embodiment of the $CO_2$ signal processing performed by the capnograph electronics **30** (or alternatively in whole or in part by a nurses' station monitor, bedside patient monitor, or other device with a suitably programmed electronic data processor) is diagrammatically shown in FIGURE 1. The $CO_2$ signal is sampled and optionally corrected for factors such as the presence of interfering gases (e.g. nitrous oxide), barometric pressure, and so forth in order to generate a capnogram **40**. The capnogram is a signal representing the partial pressure or concentration of carbon dioxide, denoted in FIGURE 2 as $[CO_2]$, as a function of time. Diagrammatic FIGURE 2 illustrates the capnogram **40** as an idealized waveform for a healthy patient, in which every breath is identical and exhibits near-zero $[CO_2]$ during the inspiratory phase and a well-defined maximum $[CO_2]$ that rises gradually over the expiratory phase and terminates in a maximum $[CO_2]$ corresponding to end-tidal $CO_2$, and in which the $etCO_2$ is the same for every breath. In practice, it will be understood that the capnogram **40** for a real patient usually deviates significantly from this idealized curve due to numerous factors such as non-uniform breathing, talking, coughing, possible chronic lung problems in the case of an ill patient, or so forth. In the capnogram of a real patient, the $etCO_2$ may vary from breath to breath. The illustrative idealized example of FIGURE 2 further assumes a constant respiration rate of 4 seconds/breath, i.e. 15 breaths per minute. As is known in the art, the resting respiration rate (RR) for a normal adult patient is typically on the order of 3-5 seconds/breath (12-20 breaths per minute), with higher RR typically observed for infants (up to about 60 breaths per minute). In a real patient, the RR is generally not constant - the RR can increase significantly due to excitement or exertion, may slow during rest periods, may stop entirely during a sleep apnea episode, and/or may generally vary significantly due to various respiratory ailments or other medical conditions.

With continuing reference to FIGURES 1 and 2, in an operation **42** at a current time $t$ the maximum $CO_2$ value over a (past) time window $W$ of duration $T_W$, is determined. The duration $T_W$, of the time window $W$ for the operation **42** is chosen to encompass several breaths. For example, in some embodiments $T_W$ has a duration of at least 30 seconds (encompassing five breaths for a patient breathing at a slow RR of 10 breaths/minute, i.e. 6 sec/breath), although shorter values are contemplated, such as for infants whose RR is higher. In some embodiments $T_W$ is in the range 30 seconds to 3 minutes inclusive. For an adult, $T_W$ may be chosen to be in the range 1 minute to 2 minutes inclusive. Setting $T_W$ longer than these illustrative upper limit values is also contemplated, and may be appropriate for example in conjunction with patients who are active or otherwise exhibit significant breath-to-breath variation in the capnogram **40**.

The time window $W$ is a sliding time window. That is, the operation **42** determining the largest $[CO_2]$ value in the time window $W$ is repeated (as indicated by repeat operation **44** of FIGURE 1) for successive current time values $t$ (and corresponding time shifts of the time window $T_W$ as diagrammatically shown in FIGURE 2) at a sampling interval $T_S$ to generate an etCO$_2$ signal **50**. The sampling interval $T_S$ for the repetition **44** is typically much larger than the $[CO_2]$ measurement interval employed by the capnograph **10**. For example, the $[CO_2]$ output by the measurement cell **20** may be sampled at 10 millisecond time intervals to generate the capnogram **40**, while the sampling interval $T_S$ is 10 seconds in illustrative FIGURE 2. On the other hand, the sampling interval $T_S$ determines the temporal resolution of the etCO$_2$ signal **50**, and so it is preferably chosen to be relatively short, and in particular is much shorter than the duration $T_W$ of the sliding time window $W$. In some embodiments, the sampling interval $T_S$ is in the range 5 seconds to 15 seconds inclusive, although longer or shorter sampling intervals are contemplated.

The loop **42, 44** thus implements a sliding window maximum operation **42, 44** in which, for each current time $t$ at which an end-tidal CO$_2$ sample is taken, the largest $[CO_2]$ value of the capnogram **40** within the time window $W(t)$ is chosen as the etCO$_2$ value for current time $t$. The output is the etCO$_2$ signal **50** which has the advantages (compared with end-tidal CO$_2$ calculated on a per-breath basis) of being both smoother and a closer approximation of the maximum alveolar CO$_2$ partial pressure. Another advantage of the etCO$_2$ signal **50** is that the etCO$_2$ samples are equally-spaced at the sampling interval $T_S$; whereas, a per-breath end-tidal CO$_2$ signal is unequally spaced in accord with the breathing intervals (although the per-breath signal can be re-sampled or otherwise post-processed to provide equally-spaced data).

This sliding window maximum processing can be represented mathematically as follows:

$$etCO_2(t) = \max([CO_2])|_{W(t)} \qquad (1)$$

where $t$ denotes time, $[CO_2]$ denotes the capnograph signal **40**, the window $W(t)$ is the following portion of the the capnogram **40**:

$$W(t) = \left\{ [CO_2]_{t-T_W}, \dots, [CO_2]_{t-1} \right\} \qquad (2)$$

and the function $\max([CO_2])|_{W(t)}$ returns the maximum carbon dioxide level over the window $W(t)$. The $etCO_2(t)$ calculation of Expression (1) is repeated at the sampling interval $T_S$, e.g. at times $t_o$, $t_o + T_S$, $t_o + 2T_S$, $t_o + 3T_S$,... using corresponding time windows $W(t_o)$, $W(t_o + T_S)$, $W(t_o + 2T_S)$, $W(t_o + 3T_S)$, ... as shown in FIGURE 2 to generate the etCO$_2$ signal **50** as a function of time with sampling interval $T_S$.

As further indicated in FIGURE 2, it will be appreciated that the first iteration of this sliding window maximum operation is delayed by a delay time $T_{delay} = T_W$ in order to generate the initial window $W_o$. If this delay is considered too long, it is contemplated to use a shorter time window for the first iteration to acquire the first sample of the etCO$_2$ signal **50** more quickly, albeit with possibly greater error due to the smaller initial window duration.

In Expression (2), the window $W(t)$ is defined to have its right (i.e. highest time value) edge one sample behind the current time $t$, but more generally a delay $D$ may optionally be employed, that is, more generally:

$$W(t) = \left\{ [CO_2]_{t-D-T_W}, \dots, [CO_2]_{t-D} \right\} \qquad (2a)$$

In the window VF(t) of Expression (2a), the delay $D = 0$ is a contemplated possibility, and may be used if a stable value for $[CO_2]_t$ is available at the time operation **42** is performed.

As noted, the etCO$_2$ signal **50** is smoothed as compared to the compared with end-tidal CO$_2$ calculated on a per-breath basis due to smoothing action of taking the maximum value over the time window $W$. However, any random noise causing an erroneously high CO$_2$ value will be captured by the sliding window maximum operation **42, 44**. In the illustrative embodiment of FIGURE 1, this is suppressed by an optional smoothing filter **52**, such as a low-pass filter, a digital mean filter, a median filter, or so forth, in order to produce a smoothed etCO$_2$ signal **54**. (Note that the smoothing operation **54** is not depicted in FIGURE 2). Additionally or alternatively, suppression of an occasional spuriously high CO$_2$ value can be suppressed by detailed construction of the $\max(\cdots)$ operation of Expression (1). For example, the $\max(\cdots)$ operation may output the second- or third-highest CO$_2$ value in the window $W$, or may output the average of the $N$ highest $[CO_2]$ values in the window $W$ (where $N$ is a low positive integer, e.g. $N \leq 4$). As yet another approach, a weak smoothing filter (not shown) may be applied to the capnograph signal **40** before applying the operation **42**. For example, this weak smoothing filter may be a moving average filter that makes the replacement $[CO_2]_n \leftarrow avg\{[CO_2]_{n-1}, [CO_2]_n, [CO_2]_{n+1}\}$.

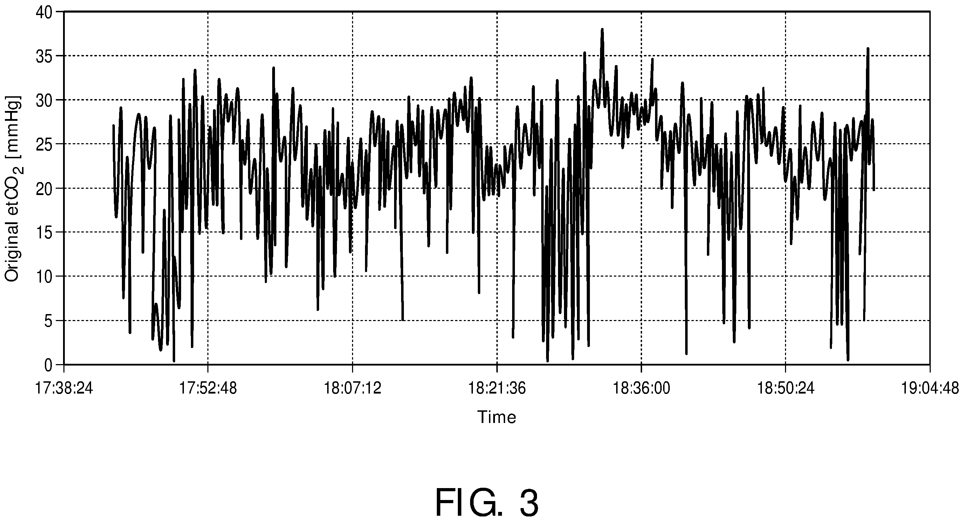

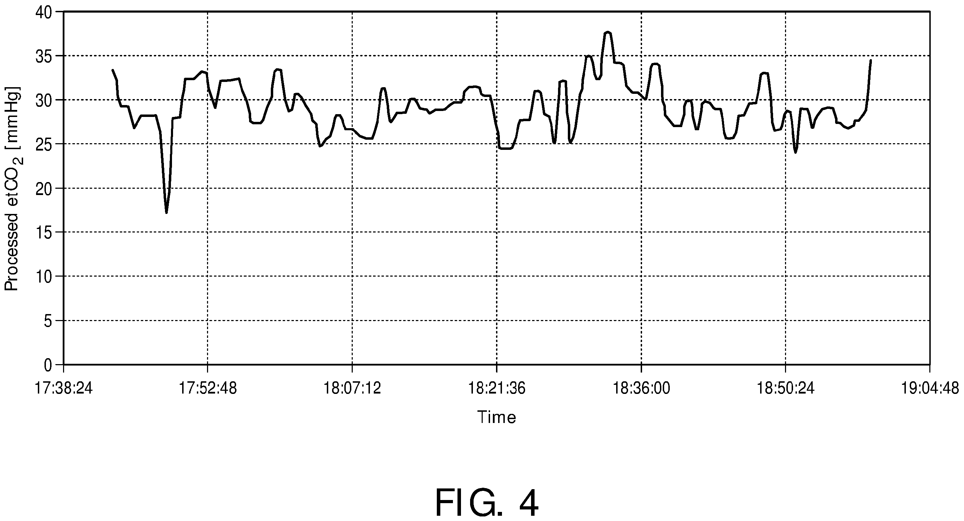

With reference to FIGURES 3 and 4, an illustrative example of the processing loop **42, 44** is shown. FIGURE 3 illustrates an experimental example of end-tidal $CO_2$ measured conventionally by taking the maximum $[CO_2]$ value over each breath. A large amount of "noise" is observed, but it will be noted that the larger-magnitude deviations making up this "noise" are mostly in the downward direction, that is, toward lower $[CO_2]$ value. This reflects the observation made herein that most clinical or physiological sources of error in end-tidal $CO_2$ (e.g. incomplete flushing of airway dead volume between breaths, parallel alveolar dead volume, impact of supplemental oxygen) tend to reduce the end-tidal $CO_2$ value produced by capnography on a per-breath basis. That is, the observed deviations are characteristic of systematic error that systematically decreases the end-tidal $CO_2$ value calculated on a per-breath basis, rather than being characteristic of true random noise.

By contrast, FIGURE 4 illustrates the $etCO_2$ signal **50** produced by applying the sliding window maximum operation **42, 44** to the same capnogram signal that was conventionally processed to produce the end-tidal $CO_2$ signal of FIGURE 3. It is seen that this experimental example of the $etCO_2$ signal **50** is much less "noisy" in that the predominantly downward deviations are removed, and the $etCO_2$ value is higher overall than the per-breath end-tidal $CO_2$ signal of FIGURE 3. The $etCO_2$ signal **50** produced by the sliding window maximum operation **42, 44** is thus a better surrogate for the alveolar maximum $CO_2$ partial pressure as compared with the end-tidal $CO_2$ data of FIGURE 3.

The invention has been described with reference to the preferred embodiments. Modifications and alterations may occur to others upon reading and understanding the preceding detailed description. The invention, however, is defined by the appended claims.

**Claims**

1.   A capnograph device (10) comprising:

a carbon dioxide measurement component (20) configured to measure respiratory carbon dioxide level; and
an electronic processor (30) programmed to:

generate a capnogram signal (40) comprising respiratory carbon dioxide level measured by the carbon dioxide measurement component as a function of time; and
compute an end-tidal carbon dioxide, etCO2, signal (50, 54) as a function of time by operations including performing a sliding window maximum operation (42, 44) on the capnograph signal wherein the sliding window maximum operation (42, 44) employs a sliding time window ($W$) encompassing several breaths; **characterized in that** the electronic processor is programmed to compute the end-tidal carbon dioxide, etCO2, signal (50,54) as the maximum value of the capnograph signal within the sliding time window (W).

2.   The capnograph device (10) of claim 1 wherein the sliding window maximum operation (42, 44) employs the sliding time window ($W$) whose duration ($T_W$) encompasses at least five breaths.

3.   The capnograph device (10) of claim 1 wherein the sliding window maximum operation (42, 44) employs the sliding time window ($W$) whose duration ($T_W$) is at least 30 seconds.

4.   The capnograph device (10) of claim 1 wherein the sliding window maximum operation (42, 44) employs the sliding time window ($W$) whose duration ($T_W$) is between one minute and two minutes inclusive.

5.   The capnograph device (10) of any one of claims 1-4 wherein the sliding window maximum operation (42, 44) employs a sampling interval ($T_S$) of between five seconds and fifteen seconds inclusive.

6.   The capnograph device (10) of any one of claims 1-5 wherein the sliding window maximum operation (42, 44) comprises computing the etCO2 signal (50) as:

$$etCO_2(t) = \max([CO_2])|_{W(t)}$$

where $t$ denotes time, $[CO_2]$ denotes the capnogram signal (40), and $W(t)$ denotes the sliding time window ($W$) as:

$$W(t) = \{[CO_2]_{t-D-T_W}, \dots, [CO_2]_{t-D}\}$$

where $D$ is a delay value and $D \geq 0$.

7. The capnograph device (10) of any one of claims 1-5 wherein performing the sliding window maximum operation (42, 44) comprises computing $etCO_2(t) = \max([CO_2])|_{W(t)}$ where $t$ denotes time, $[CO_2]$ is the capnogram signal (40) and $W(t)$ is a sliding time window.

8. The capnograph device (10) of any one of claims 1-7 wherein the electronic processor (30) is programmed to compute the etCO2 signal (50) as a function of time by operations further including applying a smoothing filter to the capnograph signal (40) prior to performing the sliding window maximum operation (42, 44) on the capnograph signal.

9. The capnograph device (10) of any one of claims 1-8 wherein performing the sliding window maximum operation (42, 44) computes an unsmoothed etCO2 signal (50) and the electronic processor (30) is programmed to compute a smoothed etCO2 signal (54) as a function of time by applying a smoothing filter (52) to the unsmoothed etCO2 signal (50).

10. The capnograph device (10) of any one of claims 1-9 further comprising: a display component (32) configured to display the etCO2 signal (50, 54).

11. A non-transitory storage medium storing instructions readable and executable by an electronic processor (30) to perform a capnography method comprising:

generating a capnogram signal (40) comprising respiratory carbon dioxide level measured by a carbon dioxide measurement component (20) as a function of time; and
performing a sliding window maximum operation (42, 44) on the capnograph signal to compute an end-tidal carbon dioxide, etCO2, signal (50) as a function of time wherein the sliding window maximum operation (42, 44) employs a sliding time window ($W$) that encompasses several breaths;
**characterized in that** the electronic processor is programmed to compute the end-tidal carbon dioxide, etCO2, signal (50,54) as the maximum value of the capnograph signal within the sliding time window (W).

12. The non-transitory storage medium of claim 11 wherein the sliding window maximum operation (42, 44) employs the sliding time window ($W$) whose duration ($T_W$) is at least 30 seconds.

13. The non-transitory storage medium of claim 11 wherein the sliding window maximum operation (42, 44) employs the sliding time window ($W$) whose duration ($T_W$) is at least one minute.

14. The non-transitory storage medium of any one of claims 11-13 wherein performing the sliding window maximum operation (42, 44) to compute the etCO2 signal (50) as $etCO_2(t) = \max([CO_2])|_{W(t)}$ a function of time comprises computing $etCO_2(t) = \max([CO_2])|_{W(t)}$ where $t$ denotes time, $[CO_2]$ is the capnogram signal (40) and $W(t)$ is a sliding time window.

15. The non-transitory storage medium of claim 14 wherein $\max([CO_2])|_{W(t)}$ returns the maximum $[CO_2]$ value over the time window $W(t)$ defined as one of:

(i) the largest capnogram signal sample over the time window W(t).

**Patentansprüche**

1. Kapnographgerät (10), umfassend:

eine Kohlendioxid-Messkomponente (20), die so konfiguriert ist, dass sie den Kohlendioxidgehalt der Atemwege misst; und
einen elektronischen Prozessor (30), der programmiert ist:

Erzeugen eines Kapnogrammsignals (40), das den von der Kohlendioxidmesskomponente gemessenen respiratorischen Kohlendioxidspiegel als Funktion der Zeit umfasst; und
Berechnen eines endtidalen Kohlendioxid, etCO2-Signals (50, 54) als Funktion der Zeit durch Operationen, einschließlich der Durchführung einer Schiebefenster-Maximaloperation (42, 44) am Kapnographensignal, wobei die Schiebefenster-Maximumbetrieb (42, 44) verwendet ein gleitendes Zeitfenster (*W*), das mehrere Atemzüge umfasst;

**dadurch gekennzeichnet, dass** der elektronische Prozessor so programmiert ist, dass er das endexspiratorische Kohlendioxid, etCO2-Signal (50,54) als Maximalwert des Kapnographensignals innerhalb des gleitenden Zeitfensters (W) berechnet.

**2.** Kapnographgerät (10) nach Anspruch 1, wobei der Schiebefenster-Maximumbetrieb (42, 44) das Schiebezeitfenster (W) verwendet, dessen Dauer ($T_W$) mindestens fünf Atemzüge umfasst.

**3.** Kapnographgerät (10) nach Anspruch 1, wobei der Schiebefenster-Maximumbetrieb (42, 44) das Schiebezeitfenster (W) verwendet, dessen Dauer ($T_W$) mindestens 30 Sekunden beträgt.

**4.** Kapnographgerät (10) nach Anspruch 1, wobei der Schiebefenster-Maximumbetrieb (42, 44) das Schiebezeitfenster (W) verwendet, dessen Dauer ($T_W$) zwischen einer Minute und einschließlich zwei Minuten beträgt.

**5.** Kapnographgerät (10) nach einem der Ansprüche 1-4, wobei der Schiebefenster-Maximumbetrieb (42, 44) ein Abtastintervall ($T_S$) zwischen fünf und fünfzehn Sekunden einschließlich verwendet.

**6.** Kapnographgerät (10) nach einem der Ansprüche 1-5, wobei der Schiebefenster-Maximumbetrieb (42, 44) die Berechnung des etCO2-Signals (50) umfasst als: $etCO_2(t)=\max([CO_2])|W_{(t)}$ wobei bezeichnet $t$ die Zeit, $[CO_2]$ das Kapnogrammsignal (40) und $W(t)$ das gleitende Zeitfenster (W) als: $W(t)=\{[CO_2]_{t-D-Tw},...[CO_2]_{t-D}\}$ wobei $D$ ein Verzögerungswert und $D{\geq}0$ ist.

**7.** Kapnographgerät (10) nach einem der Ansprüche 1-5, wobei das Durchführen der Schiebefenster-Maximum Operation (42, 44) das Rechnen umfasst $etCO_2(t)=\max([CO_2])|w_{(t)}$ wobei $t$ die Zeit bezeichnet, $[CO2]$ das Kapnogrammsignal (40) und $W(t)$ ein gleitendes Zeitfenster ist.

**8.** Kapnographgerät (10) nach einem der Ansprüche 1-7, wobei der elektronische Prozessor (30) so programmiert ist, dass er das etCO2-Signal (50) als Funktion der Zeit berechnet, und zwar durch Vorgänge, die außerdem die Anwendung eines Glättungsfilters auf das Kapnographsignal umfassen (40) vor der Durchführung des Schiebefenster-Maximumbetriebs (42, 44) am Kapnographensignal.

**9.** Kapnographengerät (10) nach einem der Ansprüche 1-8, wobei durch die Ausführung des Schiebefenster Maximumbetriebs (42, 44) ein ungeglättetes etCO2-Signal (50) berechnet wird und der elektronische Prozessor (30) so programmiert ist, dass er ein geglättetes etCO2 berechnet Signal (54) als Funktion der Zeit durch Anwenden eines Glättungsfilters (52) auf das ungeglättete etCO2-Signal (50).

**10.** Kapnographgerät (10) nach einem der Ansprüche 1-9, ferner umfassend: eine Anzeigekomponente (32), die zum Anzeigen des etCO2-Signals (50, 54) konfiguriert ist.

**11.** Nichtflüchtiges Speichermedium, das von einem elektronischen Prozessor (30) lesbare und ausführbare Anweisungen speichert, um ein Kapnographieverfahren durchzuführen, umfassend:

Erzeugen eines Kapnogrammsignals (40), das den von einer Kohlendioxidmesskomponente (20) gemessenen respiratorischen Kohlendioxidspiegel als Funktion der Zeit umfasst; und Durchführen einer Schiebefenster-Maximaloperation (42, 44) am Kapnographensignal, um ein endtidales Kohlendioxid, etCO2-Signal (50) als Funktion der Zeit zu berechnen, wobei die Schiebefenster-Maximumoperation (42, 44) eine Schiebezeit verwendet Fenster (W), das mehrere Atemzüge umfasst;
**dadurch gekennzeichnet, dass** der elektronische Prozessor so programmiert ist, dass er das endexspiratorische Kohlendioxid, etCO2-Signal (50,54) als Maximalwert des Kapnographensignals innerhalb des gleitenden Zeitfensters (W) berechnet.

**12.** Nichtflüchtiges Speichermedium nach Anspruch 11, wobei der Schiebefenster-Maximumbetrieb (42, 44) das Schiebezeitfenster (W) verwendet, dessen Dauer ($T_W$) mindestens 30 Sekunden beträgt.

**13.** Nichtflüchtiges Speichermedium nach Anspruch 11, wobei der Schiebefenster-Maximumbetrieb (42, 44) das Schiebezeitfenster (W) verwendet, dessen Dauer ($T_W$) mindestens eine Minute beträgt.

**14.** Nichtflüchtiges Speichermedium nach einem der Ansprüche 11-13, wobei das Ausführen der Schiebefenster-Maximum-Operation (42, 44) zur Berechnung des etCO2-Signals (50) als Funktion der Zeit die Berechnung von $etCO_2(t)=max([CO_2])|w_{(t)}$ wobei $t$ die Zeit bezeichnet, $[CO_2]$ das Kapnogrammsignal (40) und $W(t)$ ein gleitendes Zeitfenster ist.

15. Nichtflüchtiges Speichermedium nach Anspruch 14, wobei $max([CO_2])|w_{(T)}$ den Maximalwert $[CO_2]$-Wert über das Zeitfenster $W(t)$ zurückgibt, das als eines von Folgendem definiert ist:

(i) die größte Kapnogramm-Signalprobe über das Zeitfenster W(t).

**Revendications**

1. Dispositif capnographe (10) comprenant:

un composant de mesure de dioxyde de carbone (20) configuré pour mesurer le niveau de dioxyde de carbone respiratoire; et
un processeur électronique (30) programmé pour:

générer un signal de capnogramme (40) comprenant le niveau de dioxyde de carbone respiratoire mesuré par le composant de mesure de dioxyde de carbone en fonction du temps; et
calculer un signal de dioxyde de carbone, etCO2, de fin d'expiration (50, 54) en fonction du temps par des opérations comprenant l'exécution d'une opération maximale de fenêtre glissante (42, 44) sur le signal du capnographe, l'opération maximale de fenêtre glissante (42, 44) utilise une fenêtre temporelle glissante (W) englobant plusieurs respirations;
**caractérisé en ce que** le processeur électronique est programmé pour calculer le signal de dioxyde de carbone de fin d'expiration, etCO2, (50, 54) comme valeur maximale du signal du capnographe dans la fenêtre de temps glissante (W).

2. Dispositif capnographe (10) selon la revendication 1, dans lequel le fonctionnement maximum à fenêtre glissante (42, 44) utilise la fenêtre temporelle glissante (W) dont la durée ($T_W$) englobe au moins cinq respirations.

3. Dispositif capnographe (10) selon la revendication 1, dans lequel le fonctionnement maximum à fenêtre glissante (42, 44) utilise la fenêtre temporelle glissante (W) dont la durée ($T_W$) est d'au moins 30 secondes.

4. Dispositif capnographe (10) selon la revendication 1, dans lequel le fonctionnement maximum à fenêtre glissante (42, 44) utilise la fenêtre temporelle glissante (W) dont la durée ($T_W$) est comprise entre une minute et deux minutes incluses.

5. Dispositif capnographe (10) selon l'une quelconque des revendications 1-4, dans lequel le fonctionnement maximum à fenêtre glissante (42, 44) utilise un intervalle d'échantillonnage ($T_S$) compris entre cinq secondes et quinze secondes incluses.

6. Dispositif capnographe (10) selon l'une quelconque des revendications 1-5, dans lequel l'opération maximale de fenêtre glissante (42, 44) comprend le calcul du signal etCO2 (50) comme suit:

$etCO_2(t)=max([CO_2])|W_{(t)}$ où $t$ désigne le temps, $[CO_2]$ désigne le signal du capnogramme (40) et $W(t)$ désigne la fenêtre temporelle glissante (W) comme:

$$W(t) = \{ [CO_2]_{t-D-Tw}, \ldots [CO_2]_{t-D} \}$$

où $D$ est une valeur de retard et $D \geq 0$.

7. Dispositif capnographe (10) selon l'une quelconque des revendications 1-5, dans lequel l'exécution de l'opération maximale de fenêtre glissante (42, 44) comprend le calcul $etCO_2(t)=max([CO_2])|w_{(t)}$ où $t$ désigne le temps, $[CO_2]$ est le signal du capnogramme (40) et $W(t)$ est une fenêtre temporelle glissante.

8. Dispositif capnographe (10) selon l'une quelconque des revendications 1-7 dans lequel le processeur électronique (30) est programmé pour calculer le signal etCO2 (50) en fonction du temps par des opérations comprenant en outre l'application d'un filtre de lissage au signal de capnographe (40) avant d'effectuer l'opération de fenêtre coulissante maximale (42, 44) sur le signal de capnographe.

9. Dispositif capnographe (10) selon l'une quelconque des revendications 1-8 dans lequel l'opération de fenêtre coulissante maximale (42, 44) calcule un signal etCO2 non lissé (50) et le processeur électronique (30) est programmé pour calculer un signal etCO2 lissé (54) en fonction du temps en appliquant un filtre de lissage (52) au signal etCO2 non lissé (50).

10. Dispositif capnographe (10) selon l'une quelconque des revendications 1-9, comprenant en outre: un composant d'affichage (32) configuré pour afficher le signal etCO2 (50, 54).

11. Support de stockage non transitoire stockant des instructions lisibles et exécutables par un processeur électronique (30) pour réaliser un procédé de capnographie comprenant:

générer un signal de capnogramme (40) comprenant le niveau de dioxyde de carbone respiratoire mesuré par un composant de mesure de dioxyde de carbone (20) en fonction du temps; et

effectuer une opération maximale de fenêtre glissante (42, 44) sur le signal du capnographe pour calculer un signal de dioxyde de carbone de fin d'expiration, etCO2, (50) en fonction du temps, l'opération maximale de fenêtre glissante (42, 44) employant un temps glissant fenêtre (W) qui englobe plusieurs respirations; **caractérisé en ce que** le processeur électronique est programmé pour calculer le signal de dioxyde de carbone de fin d'expiration, etCO2, (50, 54) comme valeur maximale du signal du capnographe dans la fenêtre de temps glissante (W).

12. Support de stockage non transitoire selon la revendication 11, dans lequel l'opération maximale de fenêtre glissante (42, 44) utilise la fenêtre temporelle glissante (W) dont la durée ($T_W$) est d'au moins 30 secondes.

13. Support de stockage non transitoire selon la revendication 11, dans lequel l'opération maximale de fenêtre glissante (42, 44) utilise la fenêtre temporelle glissante (W) dont la durée ($T_W$) est d'au moins une minute.

14. Support de stockage non transitoire selon l'une quelconque des revendications 11-13, dans lequel l'exécution de l'opération de maximum de fenêtre glissante (42, 44) pour calculer le signal etCO2 (50) en fonction du temps comprend le calcul de $etCO_2(t)=\max([CO_2])|_{W(t)}$ où $t$ désigne le temps, $[CO_2]$ est le signal de capnogramme (40) et $W(t)$ est une fenêtre temporelle glissante.

15. Support de stockage non transitoire selon la revendication 14, dans lequel $\max([CO_2])|_{W(T)}$ renvoie la valeur maximale $[CO_2]$ sur la fenêtre temporelle $W(t)$ définie comme l'une des suivantes:

(i) le plus grand échantillon de signal de capnogramme sur la fenêtre temporelle W(t).

10

Capnograph device

30

20

16

Capnograph electronics

CO₂ meas. cell — 20

Capnogram — 40

14

Find max CO₂ in window W — 42

12

Repeat at sampling interval T_S — 44

24

Pump — 22

etCO₂ signal — 50

32

Display component

Scavenging system — 26

Optional smoothing filter — 52

Smoothed etCO₂ signal — 54

# FIG. 1

This example: Respiratory rate = 4 sec/breath = 15 breaths/minute
Sliding window size = $T_w$ = 1.5 minute (90 seconds)
Sampling rate = $T_s$ = 10 sec/sample

FIG. 2

FIG. 3

EP 3 334 340 B1

FIG. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20140330155 A
- EP 1972356 A1